# EUROPEAN PATENT APPLICATION

(11) **EP 4 374 824 A1**
(43) Date of publication of application: **29.05.2024**
(21) Application number: 22844756.1
(22) Date of filing: 20.07.2022
(51) Int. Cl.: A61F 2/12

(54) **HYBRID OR MIXED SILICONE MAMMARY PROSTHESIS WITH SMOOTH OR MICROTEXTURED SURFACE WITH POLYURETHANE COATING**

(30) Priority: 20.07.2021 BR 202021014224 U
(71) Applicant: Favaretto, Estefano Luiz, 14170-520 Sertãozinho São Paulo (BR)
(72) Inventor: Favaretto, Estefano Luiz, 14170-520 Sertãozinho São Paulo (BR)
(74) Representative: Dos Santos Cunha, Hallane Raissa
(86) International application number: PCT/BR2022/050286
(87) International publication number: WO 2023/000068

(57) **Abstract**

Composed of cohesive silicone gel (1) that may contain the microtextured base (2) or smooth base (3), which does not cause adhesion to structures posterior to the implant and occupies the entire seating part of the prosthesis and extends in a proportion of approximately 1/4 to 1/3 of the height or total profile of the prosthesis, the entire surface of the upper part of the implant is filled with polyurethane coating (4), which provides good adherence preventing migration of this implant or the upper pole of the breast, thus stabilizing the surgical outcome of the breast with the new implant, aiming for a better and longer-lasting result, the connection of the microtextured base (2) or smooth base (3), with the polyurethane coating (4) is made through a straight-line vulcanized belt (5) or zigzag vulcanized belt (6) or other appropriate closures, which effectively and safely seal the vulcanized belt with the materials, can be applied in various shapes and varied profile.

## Description

### Technical Field

The present patent application refers to a hybrid or mixed silicone breast prosthesis with a smooth or microtextured surface with a polyurethane coating, which will be implanted in humans with the aim of promoting anatomical corrections or aesthetic improvement.

Since the proposed model here is an association of various coating surfaces, which can be implanted in the breast regions specifically in spaces classified as: subglandular, subfascial, and submuscular of the breast, where the present invention provides total versatility in the application of the prosthesis.

### State of the Art

Currently, both in Brazil and worldwide, the techniques related to the invention are well known, and all are related to breast augmentation mammoplasty. To address breast deficiencies, the healthcare professional uses the surgical process where medical devices called prostheses or breast implants are applied.

Existing for many years and bringing benefits in their use, prosthesis implants with various types of surfaces: textured, microtextured, smooth, and polyurethane, always separately. There is also the technique of placing these implants that basically use three positions in the breast region: retromuscular, retro-glandular, and retrofascial. These positions are relatively well defined, and the implants placed there can be: textured, microtextured, microtextured, and smooth, in all positions. However, the polyurethane-coated surface implant is contraindicated for use in the retro-muscular position.

### Technical Problems

The implants available in the market so far and the surgical techniques for their use are well known. Thus, we can highlight the characteristics and deficiencies of the different surface textures used.

Smooth surface: implants coated with this type of surface have low adhesion, so often the final result is compromised by the lack of fixation of the implant in the surgical site.

Microtextured surface: implants coated with this type of surface behave similarly to a smooth surface, thus the results also have little adherence for fixation in the surgical site.

Textured surface: these implants coated with this type of surface propose to have a little more adherence, consequently, they have the most predictable results regarding adaptation to the surgical site.

Polyurethane surface: implants with this type of surface propose high adherence, and represent risks for the structures behind them, as these structures depend on the surgical site and the pectoral muscle or the rib cage with noble and vital structures.

Currently, all techniques and devices used for breast augmentation surgery, mentioned above, only increase breast size, without participating in the dynamics of the region. That is, regardless of regional movements, which mostly involve the pectoral muscle, the implant will behave independently of the functions of these movements. This increases friction and trauma, potentially leading to the formation of inflammatory reactions and even unpredictable cascading effects.

When we place the existing implants, these will be related to the anterior or posterior face, depending on the position they are in relation to the pectoral muscle. Once the implant is coated with polyurethane, this surface will adhere a lot and try to block the region. If the surface is textured, microtextured, or smooth, it will remain in a position regardless of movement, and over time it can easily migrate, thus deforming the final result, in addition to having the possibility of successive inflammatory reactions.

### Proposed Solution

The main objective of this patent application is to combine physical characteristics of the two materials into a single product. The hybrid or mixed silicone breast prosthesis with a smooth or microtextured surface with polyurethane coating will give us the possibility of being safely implanted in any of the spaces, whether subglandular, subfascial, or submuscular. The microtextured base of the hybrid and mixed prosthesis prevents adhesion to the rib cage, while the majority is composed of polyurethane that will provide greater adherence preventing early breast ptosis and prosthesis migration.

The hybrid or mixed silicone breast prosthesis with a smooth or microtextured surface with polyurethane coating will be better visualized according to the attached figures:

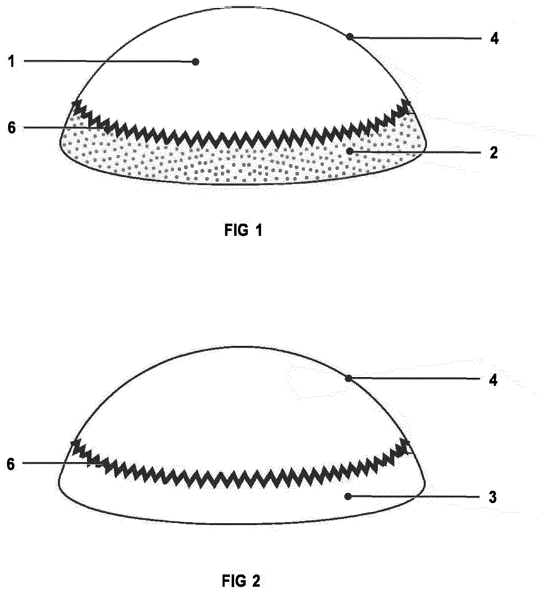

Figure 1 is a perspective view of a hybrid or mixed silicone prosthesis with a microtextured surface base and upper part with polyurethane coating with vulcanized belt between parts, in a zigzag line.
Figure 2 is a perspective view of a hybrid or mixed silicone prosthesis with a smooth surface base and upper part with polyurethane coating with vulcanized belt between parts, in a zigzag line.

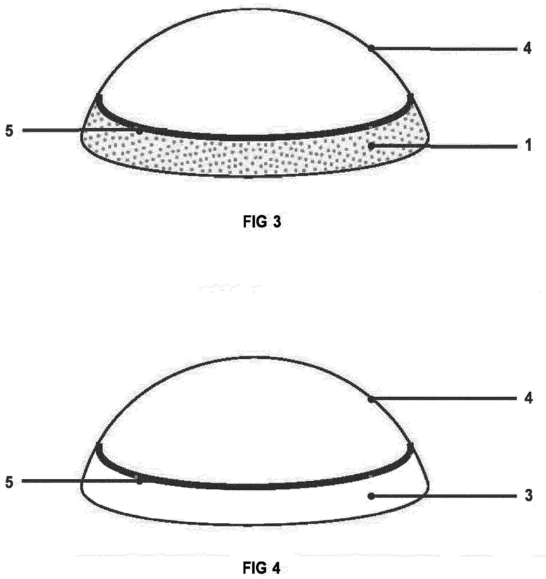

Figure 3 is a perspective view of a hybrid or mixed silicone prosthesis with a microtextured surface base and upper part with polyurethane coating with vulcanized belt between parts, in a straight line.
Figure 4 is a perspective view of a hybrid or mixed silicone prosthesis with a smooth surface base and upper part with polyurethane coating with vulcanized belt between parts, in a straight line.

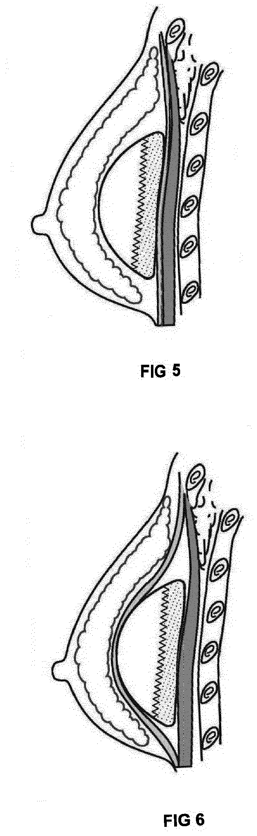

Figure 5 is a longitudinal section, showing the breast implant implanted in the subglandular space.
Figure 6 is a longitudinal section, showing the breast implant implanted in the subfascial space.

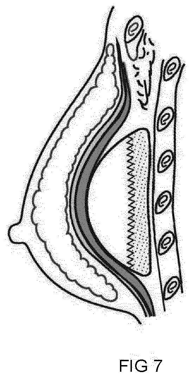

Figure 7 is a longitudinal section, showing the breast implant implanted in the submuscular space.

### Detailed Description

[1] As can be seen, the hybrid or mixed silicone breast prosthesis with a smooth or microtextured surface with polyurethane coating in question has its volume composed of cohesive silicone gel 1 that may contain the microtextured base 2 or smooth base 3, not causing adhesion to structures posterior to the implant and occupying the entire seating part of the prosthesis and extending in a proportion of approximately 1/4 to 1/3 of the total height or profile of the prosthesis. The entire surface of the upper part of the implant is filled with polyurethane coating 4, which provides good adherence avoiding migration of this implant or the upper pole of the breast, thus stabilizing the surgical outcome of the breast with the new implant, aiming for a better and longer-lasting result. The connection of the microtextured base 2 and smooth base 3, with the polyurethane coating 4, is made with a vulcanized belt in a straight line or zigzag vulcanized belt 6 or other appropriate closures, which seal the vulcanized belt of materials effectively and safely.
[2] As can be observed, the medical device proposed here can be applied in various shapes and with varied profiles, at the discretion of clinical and anatomical evaluations of the patient by the surgeon, to together choose the most suitable format and profile for each patient.
[3] Therefore, it is perceived that, while the above has been described by way of illustrative example for this patent application, all other modifications and variations made to this application, in the manner that would be apparent to those skilled in the art, are considered within the broad scope and ambit of this patent application.

## Claims

1. HYBRID OR MIXED SILICONE MAMMARY PROSTHESIS WITH SMOOTH OR MICROTEXTURED SURFACE WITH POLYURETHANE COATING. **Characterized by** being composed of cohesive silicone gel (1) that may contain the microtextured base (2) or smooth base (3), not causing adhesion to structures posterior to the implant and occupying the entire seating part of the prosthesis and extending in a proportion of approximately 1/4 to 1/3 of the height or total profile of the prosthesis, the entire surface of the upper part of the implant is filled with polyurethane coating (4), which provides good adherence preventing migration of this implant or the upper pole of the breast, thus stabilizing the surgical outcome of the breast with the new implant, aiming for a better and longer-lasting result, the connection of the microtextured base (2) and smooth base (3), with the polyurethane coating (4) is made with a straight-line vulcanized belt (5) or zigzag vulcanized belt (6) or other appropriate closures, which effectively and safely seal the vulcanized belt with the materials, can be applied in various shapes and varied profiles.
